# EUROPEAN PATENT APPLICATION

(11) **EP 4 745 224 A1**
(43) Date of publication of application: **20.05.2026**
(21) Application number: 24838834.0
(22) Date of filing: 10.07.2024
(51) Int. Cl.: C12N 1/20, C12P 13/00, A61K 35/747, A61P 25/24, A23L 33/135

(54) **LACTOBACILLUS GASSERI BDUP, USE THEREOF AND PRODUCT THEREOF**

(30) Priority: 11.07.2023 CN 202310840349
(71) Applicant: Beijing Quantihealth Technology Co., Ltd., Beijing 100160 (CN)
(72) Inventor: ZHAO, Bowen, Beijing 100070 (CN); XU, Wenyi, Beijing 100070 (CN); LIU, Yanhong, Beijing 100070 (CN); ZHANG, Huanhuan, Beijing 100070 (CN); FENG, Jinju, Beijing 100070 (CN)
(74) Representative: Stork Bamberger Patentanwälte PartmbB
(86) International application number: PCT/CN2024/104760
(87) International publication number: WO 2025/011582

(57) **Abstract**

*Lactobacillus gasseri* BDUP, the use thereof and a product thereof. The *Lactobacillus gasseri* BDUP can be used for producing GABA and preventing and/or treating depression. The *Lactobacillus gasseri* BDUP can produce GABA at a high yield under the condition of not additionally adding glutamic acid, can improve depressive behaviors of mice, and can provide potential strains for probiotic treatment for stress mood disorders such as depression and insomnia, and therefore has great potential market value.

## Description

### Technical Field

The present application relates to a field of microorganisms and their applications, and particularly relates to *Lactobacillus gasseri* BDUP and its applications.

### Background

Gamma-aminobutyric acid (GABA) is an important inhibitory neurotransmitter in the central nervous system, which exerts significant physiological regulatory effects on mammals, including lowering blood pressure, improving sleep quality, alleviating anxiety, suppressing excitation, enhancing memory and delaying neural aging. The human body has an endogenous self-regulating system for GABA. Deficiency of GABA may lead to symptoms such as irritability, restlessness, fatigue and insomnia. In the human body, the endogenous GABA level tends to decrease gradually with aging and increasing mental stress.

As the research progressed, the physiological functions of GABA have been increasingly clarified. GABA has developed into a novel functional factor and is being gradually widely applied in industries such as food, medicine, cosmetics and agriculture. Since 2001, countries around the world have successively approved GABA as a food ingredient. Therefore, appropriate exogenous supplementation of GABA can help relieve mental stress, regulate mood and improve sleep quality in populations under high pressure.

Daily supplementation of GABA is mainly achieved through direct administration of GABA supplements and daily diet. For instance, GABA is rich in foods such as tomatoes, potatoes, grapes, pumpkins as well as some fermented products including pickles and fermented brown rice. However, the natural content of GABA is relatively low, making it difficult to obtain large quantities of GABA from natural sources. At present, the main preparation methods are chemical synthesis and microbial fermentation. Nevertheless, chemically synthesized GABA is prone to raw material residues, with insufficient purity and poor safety, thus failing to meet the application requirements in the food industry. Microbial fermentation is recognized as a safe approach for large-scale production of GABA with low cost, and the GABA produced via this method can be used as a natural functional food ingredient in protective food products.

In recent years, studies on gut microbiota have revealed a strong connection between the brain and the gut, which is medically termed the gut-brain axis. Gut microorganisms can exert regulatory effects on the brain, such as influencing behavior, appetite regulation and serotonin metabolism. Alterations in gut microbiota are associated with various neurological disorders including anxiety disorders, multiple sclerosis, autism spectrum disorder and Parkinson's disease. Moreover, the homeostasis of gut microbiota also affects the expression of neurotransmitters, such as some probiotic strains in the gut, for example *Lactobacillus plantarum, Lactobacillus brevis* and *Lactobacillus pentosus.* In addition to the above effects, gut probiotics play a vital role in regulating human health. for example, maintain glycolipid metabolism balance and intestinal homeostasis. Therefore, the exploration and development of intestinal strains with high GABA-producing capacity hold promising market application prospects. These strains can not only provide highsafety GABA for patients with stress-related mood disorders such as depression and insomnia, but also exert the beneficial effects of probiotics in maintaining human health.

### Summary

To address the aforementioned problems in the prior art, the present application provides a strain of *Lactobacillus gasseri* BDUP with highefficiency GABA-producing capability.

One aspect of the present application is to provide a strain of *Lactobacillus gasseri* BDUP with the preservation number of CGMCC No. 18357.

The second aspect of the present application is to provide the use of at least one of the following preparations containing the aforementioned *Lactobacillus gasseri* BDUP in production of GABA:
1) *Lactobacillus gasseri* BDUP;
2) *Lactobacillus gasseri* BDUP bacterial agent;
3) *Lactobacillus gasseri* BDUP viable bacterial suspension;
4) *Lactobacillus gasseri* BDUP inactivated bacterial suspension;
5) *Lactobacillus gasseri* BDUP metabolite;
6) *Lactobacillus gasseri* BDUP extract.

The third aspect of the present application is to provide the use of at least one of the following preparations containing the aforementioned *Lactobacillus gasseri* BDUP in preparing a product for the prevention and/or treatment of depression:
1) *Lactobacillus gasseri* BDUP;
2) *Lactobacillus gasseri* BDUP bacterial agent;
3) *Lactobacillus gasseri* BDUP viable bacterial suspension;
4) *Lactobacillus gasseri* BDUP inactivated bacterial suspension;
5) *Lactobacillus gasseri* BDUP metabolite;
6) *Lactobacillus gasseri* BDUP extract.

The fourth aspect of the present application is to provide a product for the prevention and/or treatment of depression, comprising at least one of the following:
1) *Lactobacillus gasseri* BDUP;
2) *Lactobacillus gasseri* BDUP bacterial agent;
3) *Lactobacillus gasseri* BDUP viable bacterial suspension;
4) *Lactobacillus gasseri* BDUP inactivated bacterial suspension;
5) *Lactobacillus gasseri* BDUP metabolite;
6) *Lactobacillus gasseri* BDUP extract.

The *Lactobacillus gasseri* of the present application can produce GABA with high yields under culture conditions without additional glutamic acid supplementation. Animal experiments have demonstrated that BDUP can significantly ameliorate depressive behaviors in mice.

Deposition Instructions for the Strain of the Present Application:
Strain Name: BDUP
Latin Name: *Lactobacillus gasseri*
Taxonomic Nomenclature: *Lactobacillus gasseri*
Strain Number: 18357
Depository Institution: China General Microbiological Culture Collection Center
Abbreviation of the Depository Institution: CGMCC
Address: No. 3, Courtyard 1, West Beichen Road, Chaoyang District, Beijing
Date of Deposition: August 2, 2019
Registration Number of the Depository Center: CGMCC No. 18357.

### Brief Description of the Drawings

To illustrate the embodiments of the present application or the technical solutions in the prior art in a comprehensive manner, the accompanying drawings required for describing the embodiments or the prior art will be briefly introduced below. It is apparent that the accompanying drawings in the following description show some embodiments of the present application, and those skilled in the art can obtain other drawings based on these accompanying drawings without creative effort.
FIG.1 shows the mass spectrometry identification spectrum of *Lactobacillus gasseri* BDUP of the present application.
FIG.2 shows the Average Nucleotide Identity (ANI) analysis heatmap of BDUP of the present application and other *Lactobacillus gasseri* strains.
FIG.3 shows the effect of *Lactobacillus gasseri* BDUP of the present application on the depressive behaviors of depression model mice. (A) Statistical results of immobility time in the tail suspension test. (B) Statistical results of immobility time in the forced swimming test. n=8; vs. normal group, ***p<0.001; vs. model group, ##p<0.01, ###p<0.001.

### Detailed description of the Embodiments

### Example 1: Identification of Lactobacillus gasseri BDUP

Fecal samples were collected from a centenarian living in Hainan, China. After dilution, the samples were spread onto MRS solid medium plates (comprising 10g peptone, 10g beef extract, 5g yeast extract, 2g diammonium hydrogen citrate, 20g glucose, 1mL Tween-80, 5g sodium acetate, 2g dipotassium hydrogen phosphate, 0.58g magnesium sulfate, 0.25g manganese sulfate, 18g agar, and 1000mL distilled water). Anaerobic culture was carried out at 37°C for 24 hours, followed by isolation of distinct single colonies on the plates. Different single colonies were picked using sterilized inoculating loops and streaked onto new MRS solid medium plates for purification. After anaerobic culture at 37°C for 24 hours, purified single colonies were obtained. Each purified single colony was spread onto a mass spectrometry plate, with lysis buffer and matrix added respectively. After drying, identification was performed via mass spectrometry using a MALDI-TOF MS 1000 mass spectrometer (Autobio, Zhengzhou Autobio Diagnostics Co., Ltd.). Upon completion of identification, all strains were cryopreserved at -80°C in the strain resource library of the Applicant for subsequent use.

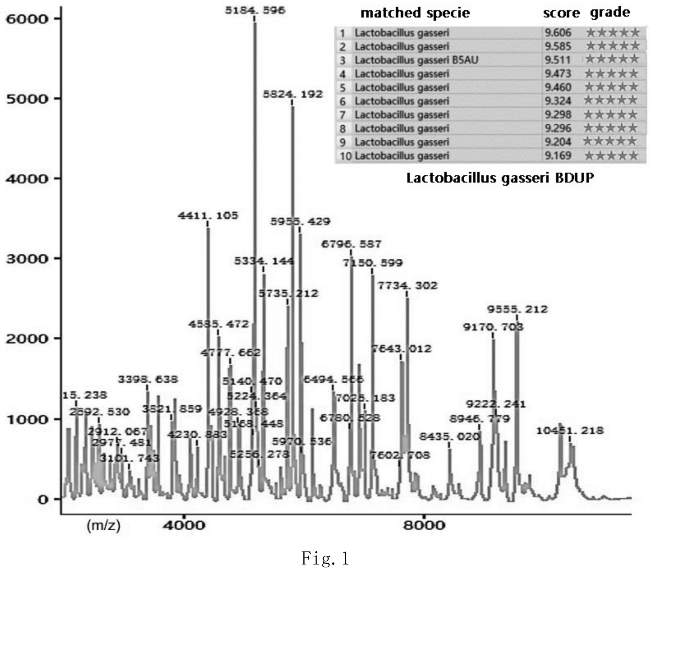

The identification results of the *Lactobacillus gasseri* BDUP strain involved in the present application are shown in FIG. 1. As indicated in FIG.1, the BDUP strain exhibits extremely high similarity to *Lactobacillus gasseri* in terms of classification, and thus, it was named *Lactobacillus gasseri* BDUP strain.

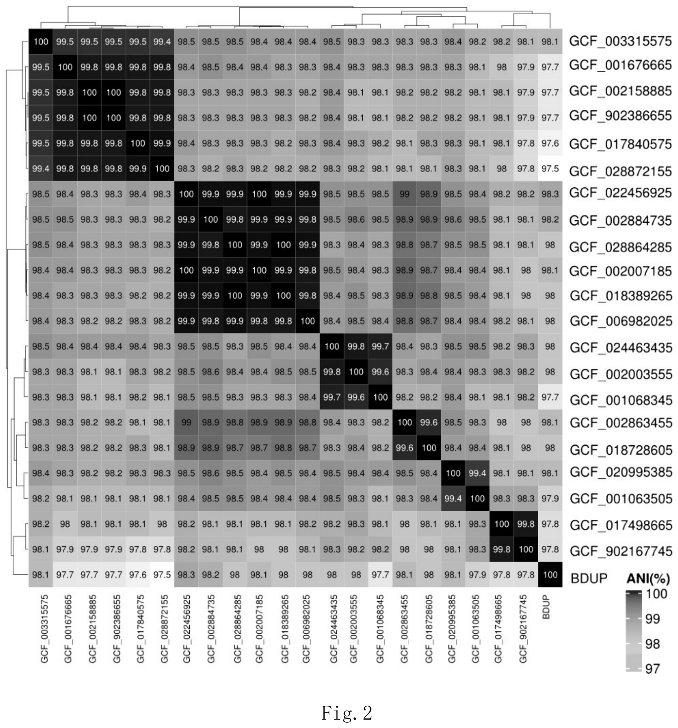

Further, the BDUP bacterial pellets of the present application were collected by centrifugation, and bacterial DNA was extracted and sent to a sequencing company. After sequencing, genome assembly was performed. Alignment of the BDUP genome information with the genomes of several *Lactobacillus gasseri* strains in the NCBI database revealed that the FastANI (Fast Average Nucleotide Identity) value of the BDUP strain of the present application is far greater than 95% (FIG.2). In identification and analysis, an ANI value of 95% is generally adopted as the species demarcation standard. Therefore, it was further confirmed that the BDUP strain of the present application belongs to the species *Lactobacillus gasseri.*

### Example 2: Preparation of Viable Bacterial Suspension and Fermentation Metabolites of Lactobacillus gasseri BDUP

The BDUP bacterial suspension stored at -80°C was spread onto MRS solid medium plates, followed by inverted incubation at 37°C for 24 hours.

Single colonies were picked and inoculated into liquid MRS medium (comprising 10g peptone, 10g beef extract, 5g yeast extract, 2g diammonium hydrogen citrate, 20g glucose, 1mL Tween-80, 5g sodium acetate, 2g dipotassium hydrogen phosphate, 0.58g magnesium sulfate, 0.25g manganese sulfate, and 1000mL distilled water), and cultured at 37°C for 24 hours to obtain a first-generation bacterial culture. A 10% volume of the first-generation bacterial culture was inoculated into fresh liquid MRS medium and cultured at 37°C for 24 hours to obtain a second-generation bacterial culture. A 10% volume of the second-generation bacterial culture was inoculated into fresh liquid MRS medium and cultured at 37°C for 24 hours to obtain a working bacterial culture. The working bacterial culture was centrifuged at 13,000 rpm and 4°C for 15 minutes. The supernatant was collected as the fermentation metabolite of the BDUP strain for subsequent experiments, while the bacterial pellet was resuspended in physiological saline to obtain a viable bacterial suspension containing live bacterial cells.

### Example 3: Detection of GABA

### (1) Paper Chromatography

1mL of the fermentation broth of *Lactobacillus gasseri* BDUP was centrifuged at 12,000 rpm and 4°C for 10 minutes to remove bacterial cells, and the supernatant was transferred to a new centrifuge tube for storage and later use. 4µL of the sample supernatant (with 2 replicates set), the corresponding medium, and GABA standard solutions of various concentrations (0.2, 0.4, 0.8, 1.2, 1.6, and 2.0 g/L) were spotted onto filter paper. The spotting line was 2.0 cm away from the edge of the chromatographic paper, and the spacing between adjacent spots was 2.0 cm. After the sample spots dried, the chromatographic paper was developed in a developing solvent [V(n-butanol): V(glacial acetic acid): V(water) = 5:3:2, with ninhydrin added at a dosage of 1.2% (w/v)] for 50 minutes (development was terminated when the developing solvent front was approximately 1 cm away from the top edge of the chromatographic paper). Upon completion of chromatography, the chromatographic paper was immediately placed in an oven at 90°C for drying and color development for 30 minutes. After color development, the spots with the same Rf value as the standard product were cut out and eluted with 5 mL of eluent [V(75% ethanol): V(0.6% copper sulfate solution) = 38:2] at 40°C and 50 rpm for 40 minutes. After elution, the eluent was thoroughly mixed. 200 µL of the mixture was transferred to an ELISA plate (with 3 replicates set), and the absorbance value was measured at a wavelength of 510 nm. The measured absorbance value (y value) was substituted into the standard curve equation plotted using the GABA standard solutions to calculate the GABA concentration in the sample (x, unit: g/L).

The GABA-producing capacity of *Lactobacillus gasseri* BDUP cultured in liquid MRS medium (without additional L-glutamic acid supplementation) for 24 hours was determined via paper chromatography for three times, and the results are shown in Table 1.

**Table 1 GABA Production of BDUP Strain Detected by Paper Chromatography**

| Detection Time | Strain | Medium | Concentration (mg/L) |
|---|---|---|---|
| 1 | BDUP | MRS | 892 |
| 2 | BDUP | MRS | 785 |
| 3 | BDUP | MRS | 841 |

### (2) HPLC Method

At present, the precolumn derivatization-HPLC method is widely adopted for the determination of GABA content. Relevant references include, but are not limited to: "Nutritional Components, Gamma-Aminobutyric Acid Content and Anti-fatigue Activity of Germinated Brown Rice Bran", "Determination of γ-aminobutyric Acid in Bean Products and Sprouted Brown Rice by OPA-derivation HPLC Method", "Detection of γ-aminobutyric Acid in Sports Drinks by Precolumn Derivatization-HPLC" and "Simultaneous Determination of Amino Acid and Monoamine Neurotransmitters in Serum by High Performance Liquid Chromatography Coupled with Precolumn Derivatization".

The GABA standard was accurately weighed to prepare standard solutions with mass concentrations of 0.04, 0.08, 0.12, 0.16, and 0.20g/L. 100µL of the GABA standard solution at each concentration was taken, and 100µL of o-phthalaldehyde (OPA) derivatizing agent was added to each standard solution, followed by thorough mixing and incubation at room temperature for 2 min. After the reaction, 20 µL of each solution was taken and measured the absorption peak value at 338 nm. A standard curve for GABA concentration determination was established using the peak area. Subsequently, o-phthalaldehyde was added to the diluted BDUP supernatant, and the mixture was incubated and mixed well for detection.

The detection conditions were as follows: detector wavelength was set at 338 nm; the chromatographic column was a C18 ODS reversed-phase column (250 mm × 4.6 mm, 5 µm) maintained at room temperature; the mobile phase consisted of 52% of 0.1 mol/L phosphate buffer solution (pH 6.0), 46% of methanol, and 2% of tetrahydrofuran. Prior to use, the mobile phase was filtered through a 0.22 µm microporous membrane and degassed by ultrasonication. A flow rate of the mobile phase is 1 mL/min.

The GABA production of *Lactobacillus gasseri* BDUP determined by high performance liquid chromatography (HPLC) for three times is shown in Table 2 below.

**Table 2 GABA Production of BDUP Strain Detected by HPLC Method**

| Experiment No. | Strain | Concentration (g/L) | Medium | Avg. GABA Concentration |
|---|---|---|---|---|
| 1 | BDUP | 0.743 | MRS | |
| 2 | BDUP | 0.642 | MRS | 0.694 |
| 3 | BDUP | 0.698 | MRS | |

Table 2 shows that the average GABA concentration detected in the three HPLC experiments was approximately 0.694 g/L. Many strains reported in the literature require the addition of exogenous glutamic acid to the culture system, whereas the BDUP strain of the present application can produce a relatively high level of GABA without exogenous stimulation.

### Example 4: Effect of BDUP on Depressive Behaviors in Chronic Stressinduced Depression Model Mice

The depression model mice established via chronic stress stimulation are the most commonly used animal models in studies focusing on screening drugs for depression treatment. Therefore, depression model mice constructed by chronic stress stimulation were used as the test subjects in the following examples.

### (1) Grouping and Establishment of Mouse Model

Thirty-two male Kunming mice, weighing approximately 20g per mouse, were purchased and acclimatized for one week before being randomly divided into 4 groups: the normal group, the model group, the BDUP viable bacteria group, and the fluoxetine hydrochloride group, with 8 mice per group. Except for the normal control group, mice in the other groups were subjected to the following stress stimuli in random order every week for 4 consecutive weeks in accordance with the Chronic Unpredictable Mild Stress (CUMS) protocol: food deprivation for 12h, restraint stress for 1h, moist bedding for 12h, water deprivation for 12h, light-dark cycle reversal for 24h, shaking stress for 1min, and electric footshock. Mice in the BDUP viable bacteria group were administered *Lactobacillus gasseri* BDUP viable bacteria at a dose of 10⁹ CFU/mL via gavage once daily for 28 days. Mice in the normal group and the model group were given an equal volume of MRS medium via gavage, while those in the positive control group were treated with fluoxetine hydrochloride (10 mg/kg) via intraperitoneal injection.

### (2) Mouse Tail Suspension Test

The mouse tail suspension test is a commonly used behavioral despair model in pharmacodynamics for evaluating the efficacy of antidepressant drugs. The changes in depressive behaviors of mice are assessed by observing the immobility time of mice during tail suspension. In the experiment, each mouse was taped at 1 cm from the tail tip and suspended upside down with the nose 20-25 cm above the ground. An animal behavior video analysis system was used to record the despair behaviors of each mouse within 6 minutes (min), and the cumulative immobility time (seconds, s) in the last 4 minutes was calculated. The mice were tested individually to avoid visual and auditory interference among them. The criterion for judging the immobility time of a suspended mouse was that the mouse ceased struggling, remained in an upside-down hanging position and stayed completely motionless.

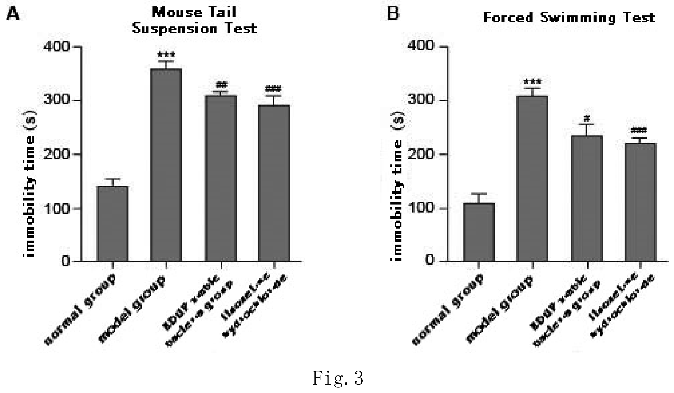

As shown in FIG.3A of the statistical results, in the tail suspension test, compared with the normal group, the immobility time of mice in the model group was significantly prolonged (p<0.001). Compared with the model group, gavage administration of viable BDUP bacteria significantly reduced the absolute immobility time of the tested mice (p<0.05), with the average immobility time decreasing from ~340 s to ~297 s. This effect was only slightly weaker than that of the drug fluoxetine hydrochloride in improving the absolute immobility time of mice (with average immobility time ~285 s).

### (3) Forced Swimming Test

The forced swimming test is an experiment used to assess the behavior of rodents for evaluating the efficacy of antidepressant drugs or foods. The improvement effect of antidepressant drugs or foods on the depressive behaviors of mice is assessed by observing the despair behaviors of mice during the experiment. In the experiment, each mouse was placed in a transparent plexiglass cylinder with a height of 20 cm and a diameter of 10 cm, filled with water at (25±1) °C to a depth of 20 cm. One mouse was tested at a time. An animal behavior video analysis system was used to record the swimming behaviors of each mouse within 6 minutes, and the cumulative immobility time (s) of the mouse during forced swimming in the last 4 minutes was calculated. The criterion for judging the immobility time of a mouse in forced swimming was that the mouse floated in the water, ceased struggling, and only exhibited slight limb movements to keep its head above the water surface.

Statistical analysis of the immobility time of mice in each group is shown in FIG.3B. Compared with the normal group, the immobility time of mice in the model group was significantly prolonged (p<0.001). *Lactobacillus gasseri* BDUP significantly reduced the absolute immobility time of the tested mice: compared with the model group, the absolute immobility time decreased from ~308 s to ~230 s, while the average absolute immobility time of the positive drug group was approximately 210 s.

A major symptom of depression is the reduction of motivated behaviors. Swimming and climbing behaviors in the forced swimming test and tail suspension test are motivated behaviors of animals, whereas absolute immobility reflects the passive and unmotivated state of animals in the experiment. Therefore, the above results indicate that the *Lactobacillus gasseri* BDUP strain of the present application can significantly alleviate the depressive behaviors of depression model mice, which provides a solid scientific basis for the future development of effective antidepressant probiotic products.

In conclusion, the *Lactobacillus gasseri* of the present application can produce GABA in high yield under culture conditions without additional glutamic acid supplementation. Three paper chromatography tests showed that the average GABA production of BDUP reached 839.3 mg/L, and animal experiments demonstrated that BDUP can significantly improve the depressive behaviors of mice.

The above descriptions are merely preferred embodiments of the present application and are not intended to limit the present application. For those skilled in the art, various modifications and variations may be made to the present application. Any modification, equivalent replacement, improvement, and the like made within the spirit and principle of the present application shall fall within the scope of protection of the present application.

## Claims

1. Use of a preparation comprising at least one of the following in production of γ-aminobutyric acid:
1) *Lactobacillus gasseri* BDUP;
2) *Lactobacillus gasseri* BDUP bacterial agent;
3) *Lactobacillus gasseri* BDUP viable bacterial suspension;
4) *Lactobacillus gasseri* BDUP inactivated bacterial suspension;
5) *Lactobacillus gasseri* BDUP metabolite;
6) *Lactobacillus gasseri* BDUP extract;
wherein the *Lactobacillus gasseri* BDUP has a preservation number of CGMCC No. 18357.

2. Use of a preparation comprising at least one of the following in preparing a product for preventing and/or alleviating depression:
1) *Lactobacillus gasseri* BDUP;
2) *Lactobacillus gasseri* BDUP bacterial agent;
3) *Lactobacillus gasseri* BDUP viable bacterial suspension;
4) *Lactobacillus gasseri* BDUP inactivated bacterial suspension;
5) *Lactobacillus gasseri* BDUP metabolite;
6) *Lactobacillus gasseri* BDUP extract;
wherein the *Lactobacillus gasseri* BDUP has a preservation number of CGMCC No. 18357.

3. A product for preventing and/or alleviating depression, comprising at least one of the following:
1) *Lactobacillus gasseri* BDUP;
2) *Lactobacillus gasseri* BDUP bacterial agent;
3) *Lactobacillus gasseri* BDUP viable bacterial suspension;
4) *Lactobacillus gasseri* BDUP inactivated bacterial suspension;
5) *Lactobacillus gasseri* BDUP metabolite;
6) *Lactobacillus gasseri* BDUP extract;
wherein the *Lactobacillus gasseri* BDUP has a preservation number of CGMCC No. 18357.

4. The product according to claim 3, **characterized in that** the product is a functional food.

5. The product according to claim 3, **characterized in that** the product is a fermented food or a protective food.
